(19) Europäisches Patentamt
European Patent Office
Office européen des brevets

(11) Veröffentlichungsnummer: **0 136 260**
**B1**

(12) **EUROPÄISCHE PATENTSCHRIFT**

(45) Veröffentlichungstag der Patentschrift: **29.04.87**

(51) Int. Cl.⁴: **C 08 G 59/40**, C 08 G 59/68

(21) Anmeldenummer: **84810375.0**

(22) Anmeldetag: **30.07.84**

(54) **Hydroxyalkyl-cyanacetate enthaltende härtbare Gemische und deren Verwendung zur Herstellung gehärteter Erzeugnisse.**

(30) Priorität: **05.08.83 CH 4267/83**

(43) Veröffentlichungstag der Anmeldung: **03.04.85 Patentblatt 85/14**

(45) Bekanntmachung des Hinweises auf die Patenterteilung: **29.04.87 Patentblatt 87/18**

(84) Benannte Vertragsstaaten: **CH DE FR GB IT LI**

(56) Entgegenhaltungen:
**FR-A-2 407 226**
**FR-A-2 454 452**

(73) Patentinhaber: **CIBA-GEIGY AG, Klybeckstrasse 141, CH-4002 Basel (CH)**

(72) Erfinder: **Renner, Alfred, Dr., Marcoup 2, CH-3280 Muntelier (CH)**

**EP 0 136 260 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft Hydroxyalkyl-cyanacetate enthaltende härtbare Gemische und deren Verwendung zur Herstellung gehärteter Erzeugnisse.

Dicyandiamid und verschiedene Cyanessigsäurederivate, vor allem Cyanessigsäureester und -amide, eignen sich bekanntlich als Härter für Epoxidharze enthaltende härtbare Gemische [vgl. z.B. US-A-4.202.920 und US-A-4.283.520]. Diese vorbekannten Cyanessigsäurederivate sind im allgemeinen lagerstabil, lassen jedoch bezüglich der Reaktivität als Härter noch zu wünschen übrig.

Mit der vorliegenden Erfindung werden lagerstabile latente Härtungsmittel für Epoxidharze mit erhöhter Reaktivität bereitgestellt.

Gegenstand der Erfindung sind härtbare Gemische, enthaltend

a) ein Epoxidharz mit durchschnittlich mehr als einer Epoxidgruppe im Molekül und

b) als Härter für das Epoxidharz eine Verbindung der Formel I

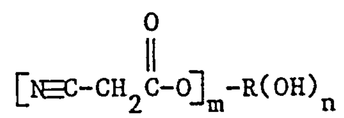 (I),

worin

n und m unabhängig voneinander eine ganze Zahl von 1 bis 5 und m und n zusammen höchstens 6 bedeuten und R einen von einem höchstens 6-wertigen Alkohol abgeleiteten Rest der Wertigkeit m, der durch Sauerstoffatome unterbrochen sein kann und ein Molekulargewicht von höchstens 200 aufweist, darstellt.

Die Verbindungen der Formel I werden zweckmässig in einer Menge eingesetzt, dass auf eine $-CH_2-CN$-Gruppe 3 bis 4 Epoxidgruppen der Komponente a) kommen.

In den Verbindungen der Formel I stellen m und n bevorzugt unabhängig voneinander die Zahl 1 oder 2 dar und m und n bedeuten zusammen 2 bis 4.

Von höchstens 6-wertigen Alkoholen abgeleitete Reste R können geradkettig oder verzweigt und definitionsgemäss durch ein oder mehrere Sauerstoffatome, besonders ein Sauerstoffatom, unterbrochen sein. Bevorzugt weisen derartige Reste 1-14 und vor allem 2-8 und bevorzugt 2-6 C-Atome auf. Geeignete Reste R sind beispielsweise:

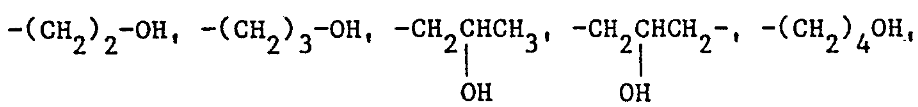

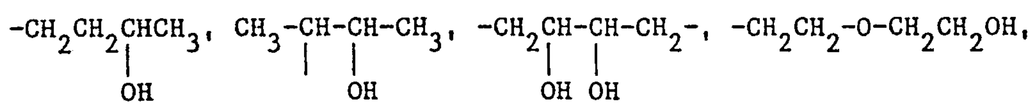

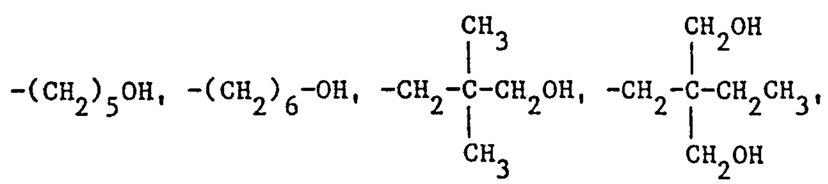

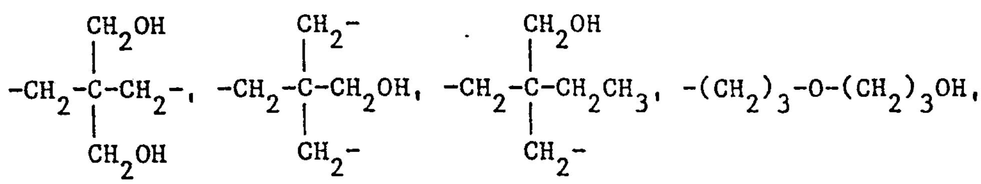

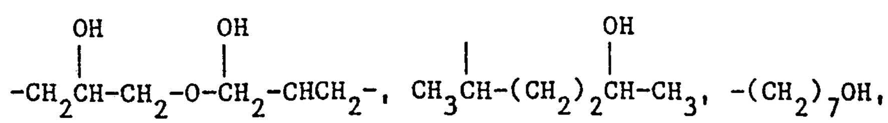

$-(CH_2)_8-OH$ $-(CH_2)_{10}OH$ und $-(CH_2)_{12}OH$, sowie

die Reste von Arabit, Xylit, D-Sorbit, D-Mannit und Dulcit.

Bevorzugt verwendet man als Komponente b) Cyanessigsäure-2-hydroxyethylester, 1,2-Propylenglykol-monocyanacetat, D-Mannit-triscyanacetat oder eine Verbindung der Formeln

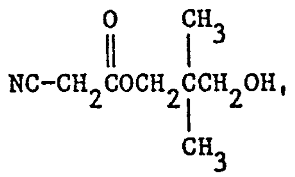, 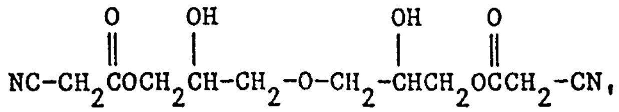,

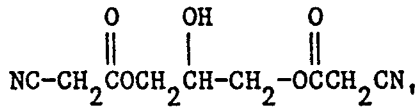, 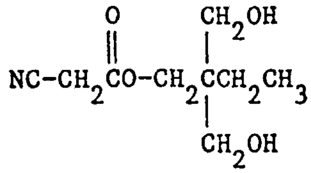 oder

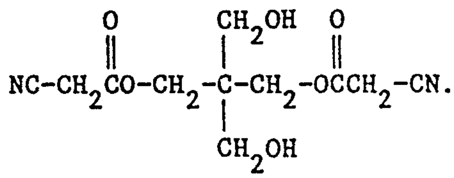.

Es können auch Gemische verschiedener Verbindungen der Formel I verwendet werden. Besonders bevorzugt verwendet man als Komponente b) 1,2-Propylenglykol-monocyanacetat.

Die Verbindungen der Formel I sind bekannt oder können auf an sich bekannte Weise hergestellt werden, z.B. durch azeotrope Veresterung von Cyanessigsäure mit entsprechenden Alkholen $R(OH)_r$, worin r eine ganze Zahl von 1 bis 6 ist und R die oben angegebene Bedeutung hat. Dabei wird die Cyanessigsäure pro Hydroxyläquivalent, das cyanacetyliert werden soll, in im wesentlichen stöchiometrischer Menge oder einem geringen Überschuss, z.B. bis etwa 1,10 Mol, eingesetzt. Die azeotrope Veresterung wird zweckmässig ohne Zusatz eines Katalysators in Gegenwart eines Schleppmittels, wie Toluol oder Xylol, in einer Konzentraion von 25-50% vorgenommen. Im allgemeinen werden nach diesem Verfahren praktisch säurefreie Lösungen der Verbindungen der Formel I erhalten. Beträgt die Säurezahl des erhaltenen Produkts mehr als 10 mg KOH/g, so wird die Reaktionslösung mit Vorteil mit einer Base, z.B. mit einer wässrigen Lösung von Natriumhydrogencarbonat, ausgeschüttelt.

Als Epoxidharze a) für die erfindungsgemässen härtbaren Gemische eignen sich insbesondere flüssige Epoxidharze, vor allem flussige aliphatische, aromatische oder heterocyclische Polyepoxide. Bevorzugt sind Polyepoxide auf Basis der mehrwertiger Phenole, besonders zweiwertiger Phenole, wie 2,2-Bis(4-hydroxyphenyl)propan (Bisphenol A), 2,2-Bis(3,5-dibrom-4-hydroxyphenyl)propan (Tetrabrom-Bisphenol A) oder 4,4'-Dihydroxydiphenylmethan (Bisphenol F); Polyepoxide auf der Basis von Novolaken, besonders Phenol-Formaldehyd- oder Kresol-Formaldehyd-Novolaken; Polyepoxide auf der Basis von mehrwertigen aliphatischen Alkholen, besonders Diolen mit 2-6 C-Atomen, wie 1,4-Butandiol; Polyepoxide auf der Basis von aromatischen Mono- oder Polyaminen, wie Aminophenolen, z.B. Triglycidyl-p-aminophenol, und N,N,N'N'-Tetraglycidyl-4,4'-diaminodiphenylmethan. Es können auch Gemische verschiedener Polyepoxide verwendet werden. Besonders bevorzugt sind flüssige, unmodifizierte Epoxidharze auf der Basis von Bisphenol F und insbesondere Bisphenol A, z.B. mit einem Epoxidgehalt von 5,1 bis 5,7 Äquivalenten/kg und einer Viskosität von etwa 7,5 bis 12,0 Pas bei 25°C.

Die erfindungsgemässen Gemische enthalten bevorzugt zusätzlich 0,1 bis 10 Gewichtsteile, bezogen auf 100 Gewichtsteile Epoxidharz a), eines Härtungsbeschleunigers c). Als Härtungsbeschleuniger c) kommen insbesondere Verbindungen der Formel II

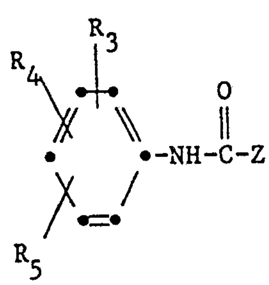 (II) in Betracht,

worin Z die Gruppe 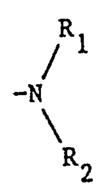 oder 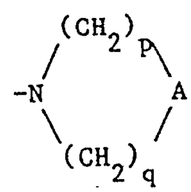 bedeutet,

wobei A für $-CH_2-$ oder -NH-, p für 0, 1 oder 2 und q für 1 oder 2 und $R_1$ und $R_2$ unabhängig voneinander für eine Alkylgruppe mit 1-4 Kohlenstoffatomen stehen, $R_3$ und $R_4$ unabhängig voneinander Wasserstoff, Halogen, Alkyl mit 1-4 Kohlenstoffatomen, Alkoxy mit 1-4 Kohlenstoffatomen, Phenyl oder Phenoxy und $R_5$ Wasserstoff, Trifluormethyl, Nitro oder eine der Gruppen

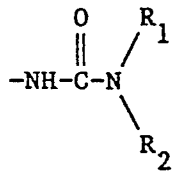 und 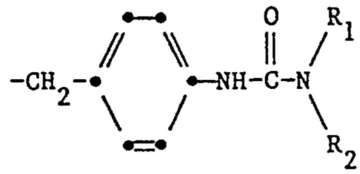 bedeuten.

Weitere geeignete Härtungsbeschleuniger c) sind Imidazole, wie 2-Methylimidazol, 2-Ethyl-4-methylimidazol, 2-Phenylimidazol und insbesondere 1-Methyl- oder 1-Ethylimidazol.

Bevorzugt verwendet man als Härtungsbeschleuniger c) Verbindungen der Formel II, in welcher Z die Gruppe $-N(R_1)(R_2)$ darstellt, $R_1$ und $R_2$ unabhängig voneinander Methyl oder Ethyl, $R_3$ und $R_4$ unabhängig voneinander Wasserstoff, Halogen, Alkyl oder Alkoxy mit 1-4 Kohlenstoffatomen und $R_5$ Wasserstoff oder Trifluormethyl bedeuten. Besonders bevorzugt ist N-(4-Trifluormethylphenyl)-N',N'-dimethylharnstoff, und ganz besonders bevorzugt sind N-(4-Chlorphenyl)-N',N'-dimethylharnstoff (= Monuron) und N-(4-Ethoxyphenyl)-N',N'-dimethylharnstoff. Der Härtungsbeschleuniger wird vorzugsweise in einer Menge von 0,1 bis 5 Gewichtsteilen, bezogen auf 100 Gewichtsteile Epoxidharz a), zugesetzt.

Zur Verbesserung bestimmter mechanischer und/oder thermischer Eigenschaften der gehärteten Produkte, z.B. der Schlagbiegefestigkeit, der Wärmestandfestigkeit und/oder der Kochwasserbeständigkeit, können den erfindungsgemässen Gemischen mit Vorteil auch Modifizierungsmittel, besonders mehrwertige Isocyanate, vor allem Diisocyanate, zugesetzt werden. Dank ihrer Bifunktionalität können die definitionsgemässen Härter b) sowohl mit Epoxiden als auch mit Isocyanaten zur Umsetzung gebracht werden. Dabei reagieren die Cyanacetylgruppen des Härters der Formel I zur Hauptsache mit dem Epoxidharz, während dessen Hydroxylgruppen vorwiegend mit dem Isocyanat reagieren. Als mehrwertige Isocyanate kommen vor allem aliphatische, cycloaliphatische, araliphatische und aromatische Di- oder Triisocyanate in Betracht. Als Beispiele geeigneter Di- und Triisocyanate seien genannt: Verbindungen der Formel $OCN-C_aH_{2a}NCO$ mit a = 4 bis 9, wie Tetramethylen- und Hexamethylendiisocyanat, Isomerengemische von 2,2,4- und 2,4,4-Trimethylhexamethylendiisocyanat; 3-Isocyanatomethyl-3,5,5-trimethyl-cyclohexylisocyanat (Isophorondiisocyanat); m- und p-Xylylendiisocyanat; aromatische Di-oder Triisocyanate, die an den aromatischen Kernen durch $C_{1-4}$-Alkylgruppen, vor allem Methylgruppen, substituiert sein können, wie m- und p-Phenylendiisocyanat, Naphthalindiisocyanat, 4,4'-Diphenyl-methandiisocyanat, 2,4- und 2,6-Tolylendiisocyanat und Triphenylmethantriisocyanat. Bevorzugt verwendet man Hexamethylendiisocyanat, 4,4'-Diphenylmethandiisocyanat oder Gemische aus 2,4- und 2,6-Tolylendiisocyanat. Die Isocyanate werden zweckmässig in einer solchen Menge eingesetzt, dass das Verhältnis der OH-Gruppen in den Verbindungen der Formel I zu den Isocyanatgruppen etwa 1:1 beträgt.

Die erfindungsgemässen Gemische können durch einfaches Zusammenführen der Komponenten und vorsichtiges Aufwärmen bis zum Lösen der Komponenten hergestellt werden. Falls ein festes Epoxidharz vorliegt, wird dieses vorübergehend zum Schmelzen erwärmt, worauf man darin den Härter und gegebenenfalls den Härtungsbeschleuniger und/oder das Modifizierungsmittel löst.

Den erfindungsgemässen Gemischen können auch Füllmittel, Streckmittel, Verstärkungsmittel, Pigmente und andere für den jeweiligen Gebrauch übliche Zusätze zugegeben werden, wie z.B. Mineralien, Holzmehl, Glas-, Kohlenstoff- oder Borfasern, Polyamide, Polyester, Russ und Metalloxide.

Die Gemische können auf den verschiedensten technischen Gebieten eingesetzt werden und z.B. als Giessharze (auch mineralisch gefüllt), z.B. für das sogenannte Druckgelierverfahren, als Spritzgussmassen, Träufelharze, Laminierharze, Klebstoffe, Formulierungen für die Oberflächenbeschichtung oder auch als Lack-Komponenten verwendet werden. Bevorzugt werden sie als Giessharze, Träufelharze oder Spritzgussmassen eingesetzt. Die erfindungsgemässen Gemische gelieren im allgemeinen bei Temperaturen zwischen 80 und 120°C. Gehärtete Produkte können dadurch hergestellt werden, dass man die Gemische auf eine Temperatur von über 120°C, bevorzugt 120 bis 200°C, erhitzt.

Die erfindungegemässen härtbaren Gemische zeichnen, sich durch eine niedrige Viskosität und vor allem durch verkürzte Gelier- und Härtungszeiten aus. Dies ist überraschend, da nicht zu erwarten war, dass Hydroxylgruppen auf die an sich bekannte Härtung von Epoxidharzen über Cyanacetylgruppen eine beschleunigende Wirkung ausüben.

In den folgenden Beispielen bedeuten Teile Gewichtsteile.

**Herstellungsbeispiele 1-8**

Beispiel 1: 1,05 Mol Cyanessigsäure werden in 30-50%iger Toluollösung bei Siedetemperatur unter azeotroper Wasserabscheidung mit 1 Mol Ethylenglykol verestert. Nach Beendigung der azeotropen

Wasserabscheidung setzt man die azeotrope Destillation noch während 3 Stunden fort, indem man das rücklaufende Toluol über gekörnten Natronkalk als Entwässerungsmittel führt. Im allgemeinen erhält man auf diese Art praktisch säurefreie Lösungen des Hydroxyalkylcyanessigesters. Beträgt die Säurezahl des erhaltenen Produkts mehr als 10 mg KOH/g, so schüttelt man die Reaktionslösung mit 5%iger $NaHCO_3$-Lösung aus. Nach dem Entfernen des Toluols am Rotationsverdampfer bei 140° C/2kPa erhält man den Cyanessigsäure-2-hydroxyethylester in einer Ausbeute von 46,4% d.Th.; Kp. 140° C/1,6 Pa, $n^D{}_{20}$ = 1,4531; Viskosität bei 20° C (gr. eta $_{20}$) = 64 mPas.

Elementaranalyse: berechnet C 46,51%, H 5,46%, N 10,85%
gefunden C 46,23% H 5,46%, N 10,90%.

Beispiel 2: Auf analoge Weise wie in Beispiel 1 beschrieben werden 1,05 Mol Cyanessigsäure mit 1 Mol 1,2-Propandiol verestert. Man erhält das 1,2-Propylenglykol-monocyanacetat in einer Ausbeute von 47,0% d.Th.; Kp. 127° C/9,3 Pa, $n^D{}_{20}$ = 1,4498; gr. eta $_{20}$ = 504 mPas.

Elementaranalyse: berechnet C 50,35%, H 6,34%, N 9,79%
gefunden C 50,16%, H 6,25%, N 10,22%.

Beispiel 3: Auf analoge Weise wie in Beispiel 1 beschrieben werden 2,10 Mol Cyanessigsäure mit 1 Mol Clycerin verestert. Man erhält das Glycerin-bis-cyanacetat der Formel

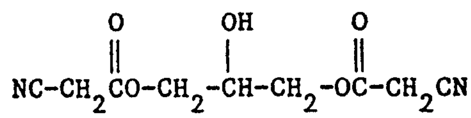

in einer Ausbeute von 94,1% d.Th. (nicht destillierbar); $n^D{}_{20}$ = 1,4774; gr. eta $_{20}$ = 80000 mPas.

Elementaranalyse: berechnet C 47,79%, H 4,46%, N 12,39%
gefunden C 47,05%, H 4,65%, N 12,15%.

Beispiel 4: Auf analoge Weise wie in Beispiel 1 beschrieben werden 2,10 Mol Cyanessigsäure mit 1 Mol Diglycerin [$HO-CH_2CH(OH)CH_2-OCH_2CH(OH)CH_2-OH$] verestert. Man erhält die Verbindung der Formel

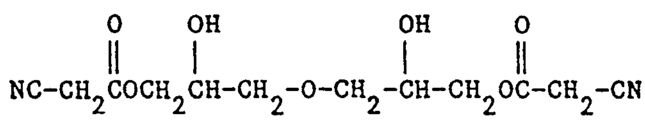

in einer Ausbeute von 95,6% d.Th. (nicht destillierbar); $n^D{}_{20}$ = 1,4830; gr. eta $_{20}$ = 60 000 mPas.

Elementaranalyse: berechnet C 48,00%, H 5,37%, N 9,33%
gefunden C 47,77%, H 5,49%, N 9,09%.

Beispiel 5: Auf analoge Weise wie in Beispiel 1 beschrieben werden 1,05 Mol Cyanessigsäure mit 1 Mol 1,1,1-Trimethylolpropan [2-Ethyl-2-(hydroxymethyl)-1,3-propandiol] verestert. Man erhält ein hellgelbes flüssiges Harz, das vorwiegend der Formel

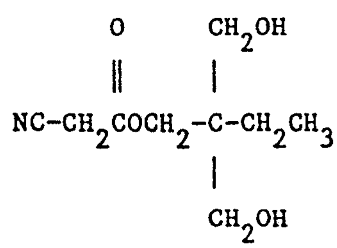

entspricht,

in einer Ausbeute von 95,3% d.Th. (nicht destillierbar); $n^D{}_{20}$ = 1,4791; gr. eta $_{20}$ = 29 100 mPas.

Elementaranalyse: berechnet C 53,72%, H 7,51%, N 6,96%,
gefunden C 53,91%, H 7,51%, N 6,85%.

Beispiel 6: Auf analoge Weise wie in Beispiel 1 beschrieben werden 2,10 Mol Cyanessigsäure mit 1 Mol 1,1,1-Trimethylolpropan verestert. Man erhält ein gelbes, zähes Harz entsprechend der Formel

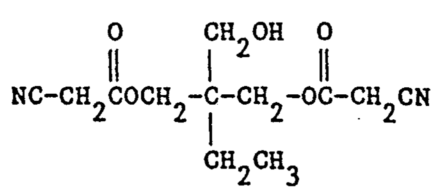

in einer Ausbeute von 95,4% d.Th. (nicht destillierbar); $n^{D}_{20}$ = 1,4793; gr. eta $_{20}$ = 72 800 mPas.

Elementaranalyse: berechnet C 53,73%, H 6,01%, N 10,44%

gefunden C 53,5%, H 6,1%, N 10,4%.

Beispiel 7: Auf analoge Weise wie in Beispiel 1 beschrieben werden 2,10 Mol Cyanessigsäure mit 1 Mol Pentaerythrit verestert. Man erhält ein bei Raumtemperatur nahezu festes Harz entsprechend der Formel

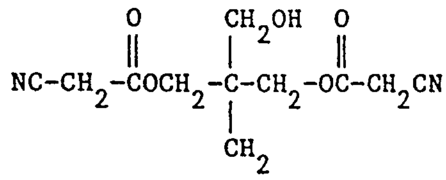

in einer Ausbeute von 93,6% d.Th; gr. eta $_{80}$ = 36200 mPas.

Elementaranalyse: berechnet C 48,89%, H 5,22%, N 10,37%

gefunden C 48,53%, H 5,36%, N 10,15%.

Beispiel 8: Auf analoge Weise wie in Beispiel 1 beschrieben werden 3,15 Mol Cyanessigsäure mit 1 Mol D-Mannit verestert. Man erhält das D-Mannit-tris-cyanacetat in einer Ausbeute von 90,6% d.Th. in Form einer harzartigen halbfesten Substanz; gr. eta $_{80}$ = 28400 mPas.

Elementaranalyse: berechnet C 47,00%, H 4,47%, N 10,96%

gefunden C 46,62%, H 4,98%, N 10,22%

**Anwendungsbeispiele I-VIII**

Beispiel I: Man stellt eine Mischung her aus 100 Teilen eines flüssigen, in Gegenwart von Natronlauge hergestellten Kondensationsproduktes aus Bisphenol A und Epichlorhydrin mit einem Epoxid-Äquivalentgewicht von 175,4 (5,7 Äquivalente/kg; Harz I), 17,22 Teilen Cyanessigsäure-2-hydroxyethylester und 2,344 Teilen N-(4-Chlorphenyl)-N',N'-dimethylharnstoff als Beschleuniger und giesst die Mischung in eine Plattenform von 150 x 150 x 4 $mm^3$. Anschliessend wird während 4 Stunden bei 100°C geliert und während je 6 Stunden bei 140°C und 180°C ausgehärtet. Man erhält eine einwandfreie zähe und harte Platte, aus der Prüfstäbe geschnitten werden. An diesen Prüfstäben werden die folgenden Eigenschaften gemessen:

Biegefestigkeit gemäss ISO 178 152 $N/mm^2$

Randfaserdehnung gemäss ISO 178 6,54%

Schlagbiegefestigkeit gemäss VSM 77105 12,3 $kJ/m^2$

Kaltwasseraufnahme nach 4 Tagen bei 25° C 0,35 Gew.%

Glasumwandlungstemperatur 102° C.

Mit dieser Mischung verklebte Aluminiumbleche weisen gemäss DIN 53283 eine Zugscherfestigkeit von 16,1 $N/mm^2$ auf.

[ISO = International Standards Organization, VSM = Verein Schweizerischer Maschinenindustrieller, DIN = Deutsche Industrienorm].

Beispiel II: 100 Teile Harz I, 20,02 Teile 1,2-Propylenglykol-mono-cyanacetat (Verbindung gemäss Beispiel 2) und 2,40 Teile N-(4-Chlor-phenyl)-N',N'-dimethylharnstoff werden wie in Beispiel 1 beschrieben gemischt, vergossen und gehärtet. Es werden die folgenden Eigenschaften ermittelt:

Biegefestigkeit (ISO 178) 143 $N/mm^2$

Randfaserdehnung (ISO 178) 5,93%

Schlagbiegefestigkeit (VSM 77105) 25,4 $kJ/m^2$

Glasumwandlungstemperatur 94° C

Kaltwasseraufnahme nach 4 Tagen bei 25° C 0,32 Gew.%

Die obige Mischung geliert in 7,67 Minuten bei 160° C.

Beispiel III: 100 Teile Harz I, 16,5 Teile Glycerin-bis-cyanacetat (Verbindung gemäss Beispiel 3) und 2,33 Teile N-(4-Chlorphenyl)-N',N'-dimethylharnstoff werden wie in Beispiel I beschrieben gemischt, vergossen und gehärtet. Es werden die folgenden Eigenschaften ermittelt:

Biegefestigkeit (ISO 178) 144,6 $N/mm^2$

Randfaserdehnung (ISO 178) 5,42%

Schlagbiegefestigkeit (VSM 77105) 20,1 $kJ/m^2$

Glasumwandlungstemperatur 116,5° C

Kaltwasseraufnahme nach 4 Tagen bei 25° C 0,355 Gew.%

Zugscherfestigkeit auf Anticorodal gemäss DIN 53283 15,8 $N/mm^2$.

Beispiel IV: 100 Teile Harz I, 21 Teile der Verbindung gemäss Beispiel 4 und 2,42 Teile N-(4-Chlorphenyl)-N',N'-dimethylharnstoff werden wie in Beispiel I beschrieben gemischt und verarbeitet. Es werden die

folgenden Eigenschaften ermittelt:
Biegefestigkeit (ISO 178) 130,3 N/mm²
Randfaserdehnung (ISO 178) 6,13%
Schlagbiegefestigkeit (VSM 77105) 35,1 kJ/m²
Wärmestandfestigkeit (ISO 175) 105°C
Kaltwasseraufnahme nach 4 Tagen bei 25°C 0,34 Gew.%
Kochwasseraufnahme nach 1 Std. bei 100°C 0,73 Gew.%
Zugscherfestigkeit auf Anticorodal (DIN 53283) 12,7 N/mm².

Beispiel V: 100 Teile Harz I, 28,14 Teile der Verbindung gemäss Beispiel 5 und 2,56 Teile N-(4-Chlorphenyl)-N',N'-dimethylharnstoff werden wie in Beispiel I beschrieben gemischt und verarbeitet. Es werden die folgenden Eigenschaften ermittelt:
Biegefestigkeit (ISO 178) 152,75 N/mm²
Randfaserdehnung (ISO 178) 5,36%
Schlagbiegefestigkeit (VSM 77105) 32,5 kJ/m²
Wärmestandfestigkeit (ISO 175) 75°C
Kaltwasseraufnahme nach 4 Tagen bei 25°C 0,33 Gew.%
Kochwasseraufnahme nach 1 Std. bei 100°C 1,33 Gew.%
Zugscherfestigkeit auf Anticorodal (DIN 53283) 13,4 N/mm².

Beispiele VIa bis VId: Diese Beispiele zeigen die Härtung von bireaktiven Gemischen aus Epoxidharz und Isocyanaten. Die Cyanacetylgruppen des Härters der Formel I reagieren mit dem Epoxidharz, während die Hydroxylgruppen vorwiegend mit dem Isocyanat reagieren. Demgemäss wird jeweils ein -OH/-NCO-Verhältis von 1 gewählt.

**Tabelle**

| Komponenten (Teile) | VIa | VIb | VIc | VId |
|---|---|---|---|---|
| Unmodifiziertes Epoxidharz auf der Basis von Bisphenol A mit einem Epoxid-Aequivalentgewicht von 190 (5,3 Aequivalente/kg) | 74,49 | 74,49 | 74,61 | 76,64 |
| Verbindung gemäss Beispiel 6 | 17,74 | 17,74 | 17,31 | 17,78 |
| Tolylen-diisocyanat (80 Gew.% 2,4- und 20 Gew.% 2,6-Derivat) | - | 5,76 | - | - |
| 4,4'-Diphenylmethandiisocyanat | - | - | 8,07 | |
| Hexamethylendiisocyanat | - | - | - | 5,57 |
| N-(4-Chlorphenyl)-N',N'-dimethylharnstoff | 1,84 | - | - | - |
| Gelierung | 8 Stunden bei 120°C | | | |
| Härtung | 8 Stunden bei 150°C | | | |
| Eigenschaften: | | | | |
| Biegefestigkeit (ISO 178)(N/mm²) | 153,4 | 130,5 | 149,3 | 150,7 |
| Randfaserdehnung (ISO 178) (%) | 5,9 | 4,0 | 6,1 | 5,4 |
| Schlagbiegefestigkeit (VSM 77105) (kJ/m²) | 18,8 | 14,1 | 22,1 | 25,3 |
| Wärmestandfestigkeit (ISO 175) (°C) | 96 | 117 | 121 | 107 |
| Kochwasseraufnahme, 1 h bei 100°C (Gew.%) | 1,01 | 0,77 | 0,72 | 0,88 |

Die Isocyanate enthaltenden gehärteten Mischungen VIc und VId zeigen gegenüber der isocyanatfreien Mischung VIa eine signifikant höhere Schlagbiegefestigkeit; die Wärmestandfestigkeiten der isocyanathaltigen Mischungen VIb, VIc un VId sind höher und deren Wasseraufnahme tiefer als bei dem isocyanatfreien Gemisch VIa.

Beispiel VII: 100 Teile Harz I, 18,9 Teile der Verbindung gemäss Beispiel 7 und 2,38 Teile N-(4-Chlorphenyl)-N',N'-dimethylharnstoff werden wie in Beispiel I beschrieben gemischt und verarbeitet. Es werden die folgenden Eigenschaften ermittelt:

Biegefestigkeit (ISO 178) 96,28 N/mm²
Randfaserdehnung (ISO 178) 3,45%
Schlagbiegefestigkeit (VSM 77105) 7,6 kJ/m²
Wärmestandfestigkeit (ISO 175) 108° C
Kaltwasseraufnahme nach 4 Tagen bei 25° C 0,36 Gew.%
Kochwasseraufnahme nach 1 h bei 100° C 0,80 Gew.%
Zugscherfestigkeit auf Anticorodal (DIN 53283) 11,15 N/mm²

Durch Zusatz von 16,53 Teilen Tolylendiisocyanat (80 Gew.% 2,4- und 20 Gew.% 2,6-Derivat) zur obigen Mischung wird die Wärmestandfestigkeit auf 144° C erhöht.

Beispiel VIII: 100 Teile Harz I, 17,87 Teile der Verbindung gemäss Beispiel 8 und 2,36 Teile N-(4-Chlorphenyl)-N',N'-dimethylharnstoff werden wie in Beispiel I angegeben gemischt und verarbeitet. Es werden die folgenden Eigenschaften ermittelt:

Biegefestigkeit (ISO 178) 121,8 N/mm²
Randfaserdehnung (ISO 178) 7,0%
Schlagbiegefestigkeit (VSM 77105) 18,4 kJ/m²
Wärmstandfestigkeit (ISO 175) 110° C
Kaltwasseraufnahme nach 4 Tagen bei 25° C 0,3 Gew.%
Kochwasseraufnahme nach 1 h bei 100° C 0,45 Gew.%
Zugscherfestigkeit auf Anticorodal (DIN 53283) 5,18 N/mm².

## Patentansprüche

1. Härtbares Gemisch, enthaltend
a) ein Epoxidharz mit durchschnittlich mehr als einer Epoxidgruppe im Molekül, und
b) als Härter für das Epoxidharz eine Verbindung der Formel

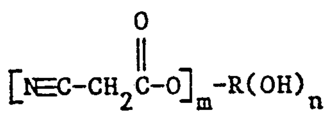 (I),

worin m und n unabhängig voneinander eine ganze Zahl von 1 bis 5 und m und n zusammen höchstens 6 bedeuten und R einen von einem höchstens 6-wertigen Alkohol abgeleiteten Rest der Wertigkeit m, der durch Sauerstoffatome unterbrochen sein kann und ein Molekulargewicht von höchstens 200 aufweist, darstellt.

2. Gemisch nach Anspruch 1, worin m und n in der Verbindung der Formel I unabhängig voneinander die Zahl 1 oder 2 und m und n zusammen 2 bis 4 bedeuten.

3. Gemisch nach Anspruch 1, das als Komponente b) Cyanessigsäure-2-hydroxyethylester, 1,2-Propylenglykol-monocyanacetat, D-Mannit-triscyanacetat oder eine Verbindung der Formeln

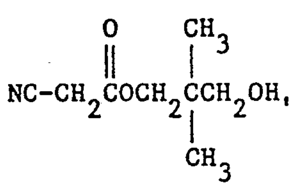, 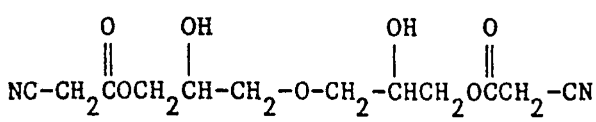,

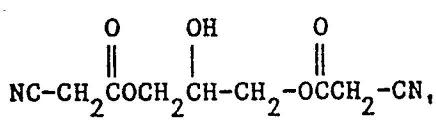, 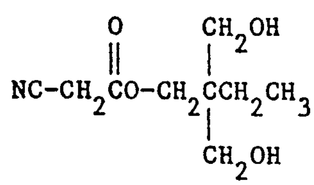 oder

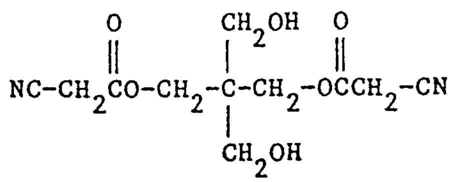 enthält.

4. Gemisch nach Anspruch 1, das als Komponente b) 1,2-Propylenglykol-monocyanacetat enthält.

5. Gemisch nach Anspruch 1, das als Komponente a) ein flüssiges Epoxidharz enthält.

6. Gemisch nach Anspruch 1, das als Komponente a) ein Epoxidharz auf Basis von Bisphenol A, Tetrabrombisphenol A oder Bisphenol F, von Phenol-Formaldehyd- oder Kresol-Formaldehyd-Novolaken, von mehrwertigen aliphatischen Alkoholen oder von aromatischen Mono- oder Polyaminen enthält.

7. Gemisch nach Anspruch 1, das als Komponente a) ein flüssiges, unmodifiziertes Epoxidharz auf der Basis von Bisphenol F und insbesondere Bisphenol A enthält.

8. Gemisch nach Anspruch 1, das zusätzlich 0,1 bis 10 Gewichtsteile, bezogen auf 100 Gewichtsteile Epoxidharz a), eines Härtungsbeschleunigers c) enthält.

9. Gemisch nach Anspruch 8, das als Härtungsbeschleuniger c) eine Verbindung der Formel II

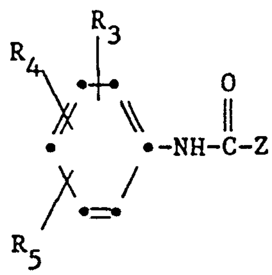 (II) enthält,

worin Z die Gruppe 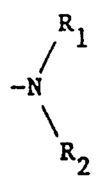 oder 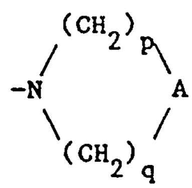 bedeutet,

A für $-CH_2-$ oder -NH-, p für 0, 1 oder 2 und q für 1 oder 2 stehen, $R_1$ und $R_2$ unabhängig voneinander eine Alkylgruppe mit 1-4 Kohlenstoffatomen, $R_3$ und $R_4$ unabhängig voneinander Wasserstoff, Halogen, Alkyl mit 1-4 Kohlenstoffatomen, Alkoxy mit 1-4 Kohlenstoffatomen, Phenyl oder Phenoxy und $R_5$ Wasserstoff, Trifluormethyl, Nitro oder eine der Gruppen

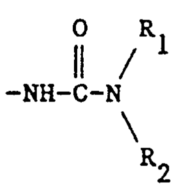 und 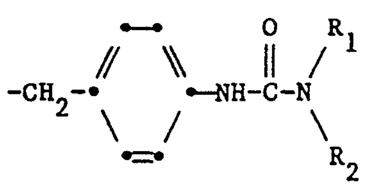 bedeuten.

10. Gemisch nach Anspruch 8 das als Härtungsbeschleuniger c) 0,1 bis 5 Gewichtsteile, bezogen auf 100 Gewichtsteile Epoxidharz a), N-(4-Trifluormethylphenyl)-N',N'-dimethylharnstoff und insbesondere N-(4-Chlorphenyl)-N',N'-dimethylharnstoff oder N-(4-Ethoxyphenyl)-N',N'-dimethylharnstoff enthält.

11. Verwendung des Gemisches nach Anspruch 1 zur Herstellung von gehärteten Produkten durch Erhitzen des Gemisches auf eine Temperatur von über 120°C.

**Claims**

1. A curable mixture containing
a) an epoxide resin having on average more than one epoxy group in the molecule, and
b) as curing agent for the epoxide resin, a compound of the formula I

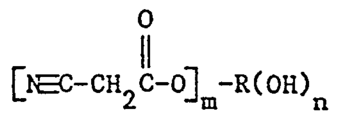

(I),

wherein
m and n are each independently of the other an integer from 1 to 5, and
m and n together are at most 6, and
R is a radical of the valency m derived from an alcohol having at most 6 hydroxyl groups, which radical can be interrupted by oxygen atoms and has a molecular weight not exceeding 200.

2. A mixture according to claim 1, wherein m and n in the compound of the formula I are each independently of the other 1 or 2, and m and n together are 2 to 4.

3. A mixture according to claim 1, which contains as component b): cyanoacetic acid-2-hydroxyethyl ester, 1,2-propylene glycol-monocyanoacetate, D-mannitol-triscyanoacetate or a compound of the formula

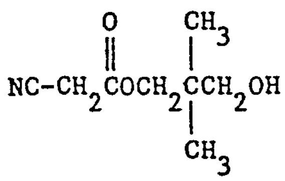, 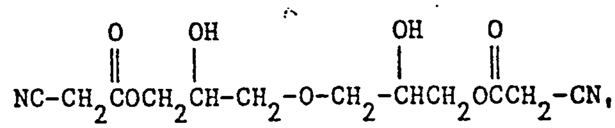,

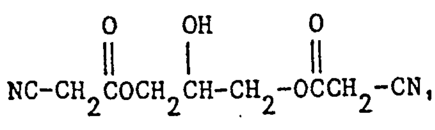, 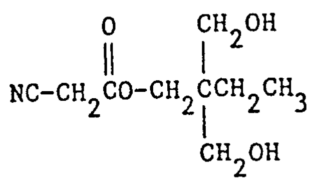 or

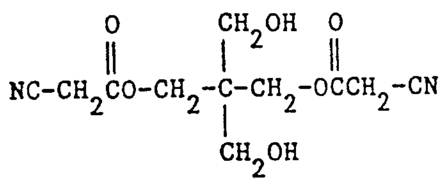

4. A mixture according to claim 1, which contains 1,2-propylene glycol-monocyanoacetate as component b).

5. A mixture according to claim 1, which contains a liquid epoxide resin as component a).

6. A mixture according to claim 1, which contains as component a): an epoxide resin based on bisphenol A, tetrabromobisphenol A or bisphenol F, on phenol-formaldehyde novolaks or cresol-formaldehyde novolaks, on polyhydric aliphatic alcohols or on aromatic mono- or polyamines.

7. A mixture according to claim 1, which contains a liquid unmodified epoxide resin based on bisphenol F or, preferably, bisphenol A, as component a).

8. A mixture according to claim 1, which additionally contains 0.1 to 10 parts by weight, based on 100 parts by weight of epoxide resin a), of a curing accelerator c).

9. A mixture according to claim 8, which contains as curing accelerator c) a compound of the formula II

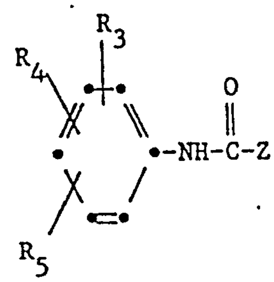

(II),

wherein Z is the group 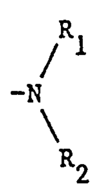 or 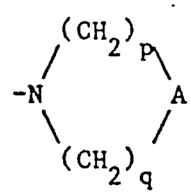,

A is $-CH_2-$ or -NH-, p is zero, 1 or 2, q is 1 or 2, $R_1$ and $R_2$ are each independently of the other an alkyl group having 1-4 carbon atoms, $R_3$ and $R_4$ are each independently of the other hydrogen, halogen, alkyl having 1-4 carbon atoms, alkoxy having 1-4 carbon atoms, phenyl or phenoxy, and $R_5$ is hydrogen, trifluoromethyl, nitro or one of the groups

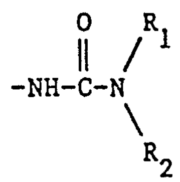 and 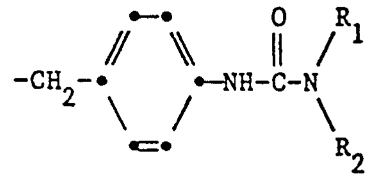 .

10. A mixture according to claim 8, which contains as curing accelerator c) 0.1 to 5 per cent by weight, based on 100 parts by weight of epoxide resin a), of N-(4-trifluoromethylphenyl)-N',N'-dimethylurea, or, preferably, N-(4-chlorophenyl)-N',N'-dimethylurea or N-(4-ethoxyphenyl)-N',N'-dimethylurea.

11. A process for producing cured products by heating a mixture according to claim 1 to a temperature of above 120°C.

**Revendications**

1. Mélange durcissable qui contient:
a) une résine époxydique renfermant en moyenne plus d'un radical époxy par molécule et
b) comme durcisseur pour la résine époxydique, un composé répondant à la formule

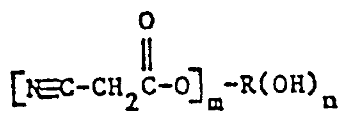 (I)

dans laquelle m et n représentent chacun, indépendamment l'un de l'autre, un nombre entier de 1 à 5, la somme (m + n) étant au plus égale à 6, et R représente un radical de valence m qui provient d'un alcool dont la fonctionnalité est au plus égale à 6, radical qui peut être interrompu par des atomes d'oxygène et qui a une masse moléculaire au plus égale à 200.

2. Mélange selon la revendication 1, dans lequel les indices m et n qui se trouvent dans la formule I représentent chacun, indépendamment l'un de l'autre, le nombre 1 ou le nombre 2, et la somme (m + n) est égale à un nombre de 2 à 4.

3. Mélange selon la revendication 1 qui contient, comme composante b), le cyanacétate d'hydroxy-2 éthyle, le monocyanacétate du propane-diol-1,2, le triscyanacétate du D-mannitol ou un composé répondant à l'une des formules suivantes

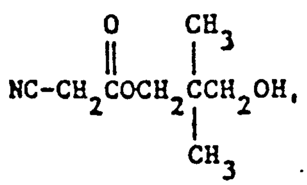 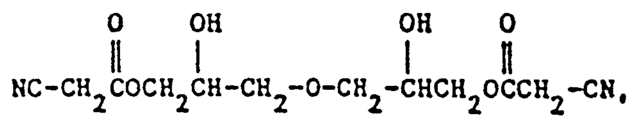

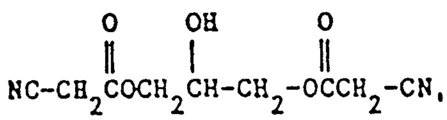 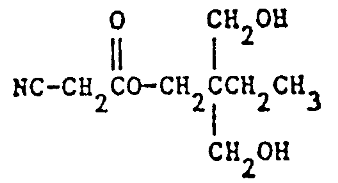 et

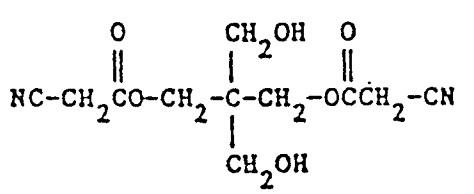

4. Mélange selon la revendication 1 qui contient, comme composante b), le monocyanacétate du propanediol-1,2.

5. Mélange selon la revendication 1 qui contient, comme composante a), une résine époxydique liquide.

6. Mélange selon la revendication 1 qui contient, comme composante a), une résine époxydique à base de bis-phénol A, de tétrabromo-bis-phénol A ou de bis-phénol F, de novolaques phénol-formaldéhyde ou crésol-formaldéhyde, de polyols aliphatiques ou de mono- ou poly-amines aromatiques.

7. Mélange selon la revendication 1 qui contient, comme composante a), une résine époxydique liquide non modifiée à base de bis-phénol F ou, mieux, de bis-phénol A.

8. Mélange selon la revendication 1 qui contient en outre de 0,1 à 10 parties en poids d'un accélérateur de durcissement c) pour 100 parties en poids de la résine époxydique a).

9. Mélange selon la revendication 8 qui contient, comme accélérateur de durcissement c), un composé répondant à la formule II

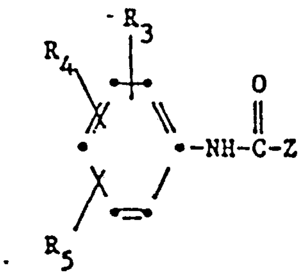

(II)

dans laquelle :

Z représente un radical 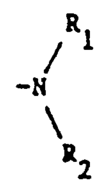 ou 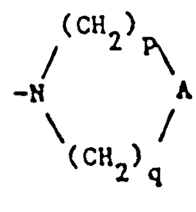

où A représente $-CH_2-$ ou -NH-, p représente un nombre égal à 0, à 1 ou à 2, q représente un nombre égal à 1 ou à 2 et $R_1$ et $R_2$ représentent chacun, indépendamment l'un de l'autre, un alkyle en $C_1$-$C_4$,

$R_3$ et $R_4$ représentent chacun, indépendamment l'un de l'autre, l'hydrogène, un halogène, un alkyle contenant de 1 à 4 atomes de carbone, un alcoxy contenant de 1 à 4 atomes de carbone, un phényle ou un phénoxy et

$R_5$ représente l'hydrogène, un trifluorométhyle, un nitro ou un radical répondant à l'une des formules suivantes:

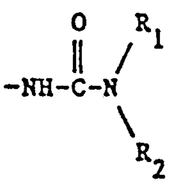 et 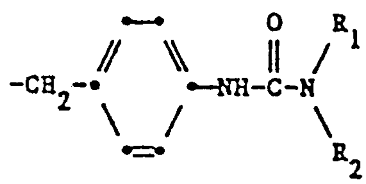.

10. Mélange selon la revendication 8 qui contient, comme accélérateur de durcissement c), de 0,1 à 5 parties en poids, pour 100 parties en poids de la résine époxydique a), de N-(trifluorométhyl-4 phényl)-N',N'-diméthyl-urée ou, plus spécialement, de N-(chloro-4 phényl)-N',N'-diméthyl-urée ou de N-(éthoxy-4 phényl)-N',N'-diméthylurée.

11. Application du mélange selon la revendication 1 à la fabrication d'objets durcis, par chauffage du mélange à une température supérieure à 120° C.